## Europäisches Patentamt

## European Patent Office

## Office européen des brevets

(11) Numéro de publication: **0 201 442 B1**

# FASCICULE DE BREVET EUROPEEN

(45) Date de publication du fascicule du brevet:
**10.01.90**

(51) Int. Cl.⁴: **A61F 2/36**

(21) Numéro de dépôt: **86460004.4**

(22) Date de dépôt: **03.04.86**

(54) **Dispositif de prothèse modulaire.**

(30) Priorité: **05.04.85 FR 8505348**

(43) Date de publication de la demande:
**17.12.86 Bulletin 86/46**

(45) Mention de la délivrance du brevet:
**10.01.90 Bulletin 90/2**

(84) Etats contractants désignés:
**AT BE CH DE GB IT LI LU NL SE**

(56) Documents cités:
**EP-A- 0 085 147**
**EP-A- 0 135 319**
**EP-A- 0 145 641**
**EP-A- 0 170 982**
**WO-A-84/03037**
**FR-A- 1 158 993**
**FR-A- 2 464 695**
**US-A- 3 918 441**

(73) Titulaire: **FRANCE IMPLANT, "La Hucheraie",
F-35360 Montauban-de-Bretagne(FR)**

(72) Inventeur: **Masse, André Abel Jean, 21, rue Brizeux,
F-35000 Rennes(FR)**
Inventeur: **Malet, Christian, Jean-Pierre, 14, rue Sarrette,
F-75014 Paris(FR)**

(74) Mandataire: **Martin, Jean-Jacques et al, Cabinet
REGIMBEAU Corre, Martin, Schrimpf, Warcoln,
Ahner 11, rue Franz Heller, F-35700 Rennes(FR)**

## Description

La présente invention concerne un dispositif de prothèse, notamment de prothèse articulaire, par exemple de prothèse de hanche (totale ou céphalique).

La mise en place de ce type de prothèse pose un problème au chirurgien car les dimensions de la prothèse qui doit être utilisée, et notamment sa largeur, dépendent des dimensions de l'os qui doit la recevoir et, en particulier, de la largeur du canal médullaire de l'os.

C'est pourquoi le bloc chirurgical doit avoir en permanence à sa disposition un stock important de prothèses de différentes largeurs échelonnées. L'immobilisation et la gestion de ce stock de pièces coûteuses, qui doit être maintenu en outre à l'état stérile, constitue une charge financière élevée pour les établissements hospitaliers ; de plus, le choix et l'utilisation des prothèses à partir d'un tel stock est peu pratique pour le chirurgien.

Il a déjà été proposé, par le document EP-A-85 147, une prothèse d'articulation de la hanche - destinée à être implantée dans l'extrémité proximale du fémur - qui est constituée de deux parties indépendantes, un corps principal ou spatule et une pièce latérale cunéiforme ; cette dernière est adaptée pour coulisser dans un profil de guidage formé dans la face latérale étroite de la spatule.

Ce dispositif connu permet apparemment de résoudre le problème qui vient d'être posé ; en effet, il est prévu dans le document EP-A-85 147 qu'on puisse disposer d'une série de pièces latérales de différentes épaisseurs, qui peuvent être combinées avec une même spatule afin de couvrir différentes largeurs de prothèse. Il est essentiellement destiné à permettre la mise en place de la prothèse à une hauteur bien déterminée dans l'extrémité du fémur. A cet effet, son implantation se fait en deux étapes :
- mise en place de la spatule dans le canal médullaire, dans la position souhaitée ;
- mise en place de la pièce latérale contre la spatule et enfoncement de cette pièce dans le fémur (tandis que la spatule est retenue dans la position souhaitée) jusqu'à ce que son bord externe cunéiforme vienne porter contre le tissu osseux et, par effet de coin, réalise le blocage de l'ensemble.

Dans ce dispositif, le corps principal de la prothèse et la pièce latérale cunéiforme sont donc deux éléments initialement séparés, qui sont accolés l'un à l'autre seulement au cours de l'implantation de la prothèse. Ces éléments ne sont jamais véritablement solidaires l'un de l'autre si bien que leur manipulation par le chirurgien, avant et pendant l'implantation, est relativement délicate et source d'imprécisions. En outre, après l'implantation, les efforts se développant sur la prothèse - qui peuvent être très élevés et de directions variables - risquent de provoquer progressivement un jeu relatif entre les deux pièces et d'entraîner un descellement de l'ensemble.

L'invention vise à remédier à ces inconvénients en proposant un dispositif de prothèse du genre énoncé dont la manipulation avant et pendant l'implantation soit aisée, et dont la retenue par l'os après implantation soit parfaite et durable.

Ces résultats sont atteints, conformément à l'invention, par le fait que le dispositif de prothèse comprend un corps de prothèse de forme allongée et une série de barrettes modulaires d'épaisseurs différentes adaptées pour constituer avec le corps de prothèse un ensemble de largeur variable, et que chacune de ces barrettes présente un bord ou une languette apte à être emboîté dans une rainure de section complémentaire ménagée latéralement dans le corps de prothèse, cet emboîtement assurant la solidarisation mutuelle, en direction latérale, de ce corps avec la barrette.

On comprendra aisément qu'il suffit ainsi que le bloc chirurgical ait à sa disposition un corps de prothèse unique, et un jeu complet de barrettes modulaires, pour pouvoir faire face à toutes les éventualités.

De plus, l'emboîtement de la barrette sélectionnée dans le corps principal est effectué avant que commence l'implantation du dispositif, ce qui facilite considérablement le maniement de l'ensemble au cours de cette opération. Après mise en place, la dissociation des deux parties, par écartement latéral, est impossible.

Chacune des barrettes présente avantageusement une épaisseur qui est sensiblement constante de l'une à l'autre de ses extrémités ; on obtient ainsi une bonne répartition des efforts d'appui avec le tissu osseux sur l'ensemble du dispositif, ce qui diminue encore le risque de descellement.

Dans un mode de réalisation préférentiel de l'invention, la languette des barrettes en queue-d'aronde et la rainure de section complémentaire du corps de prothèse ont une largeur qui varie légèrement de l'une à l'autre de leurs extrémités.

On réalise ainsi un auto-blocage de la queue-d'aronde dans la rainure associée au cours de sa mise en place (par effet de cônes auto-bloquants).

Dans ce cas, il est avantageux de prévoir une largeur qui augmente de l'extrémité proximale vers l'extrémité distale, la barrette sélectionnée étant emboîtée de bas en haut dans le corps de prothèse. Ainsi, durant l'enfoncement de la prothèse dans le canal médullaire, les frottements du tissu osseux contre les parois de la barrette tendent à la faire remonter en améliorant encore son auto-blocage dans la rainure. Après mise en place, la barrette est complètement solidaire, à la fois dans les directions latérale et longitudinale, du corps de prothèse.

L'invention sera mieux comprise, et ses particularités et avantages apparaîtront de la description et des dessins annexés, qui en représentent un mode de réalisation préférentiel.

Les figures 1 et 2 sont respectivement des vues de face et de droite du corps de prothèse seul ;

La figure 3 est une section horizontale selon le plan III-III de la figure 1 ;

La figure 4 est une vue de face du corps de prothèse auquel on a fixé une barrette modulaire de faible épaisseur ;

La figure 5 est une section horizontale suivant le plan V-V de la figure 4 ;

Les figures 6 et 8 sont des vues de face de barrettes modulaires de moyenne et de grande épaisseurs respectivement ;

Les figures 7 et 9 sont des coupes analogues à la figure 5, dans lesquelles le dispositif est équipé respectivement des barrettes des figures 6 et 8.

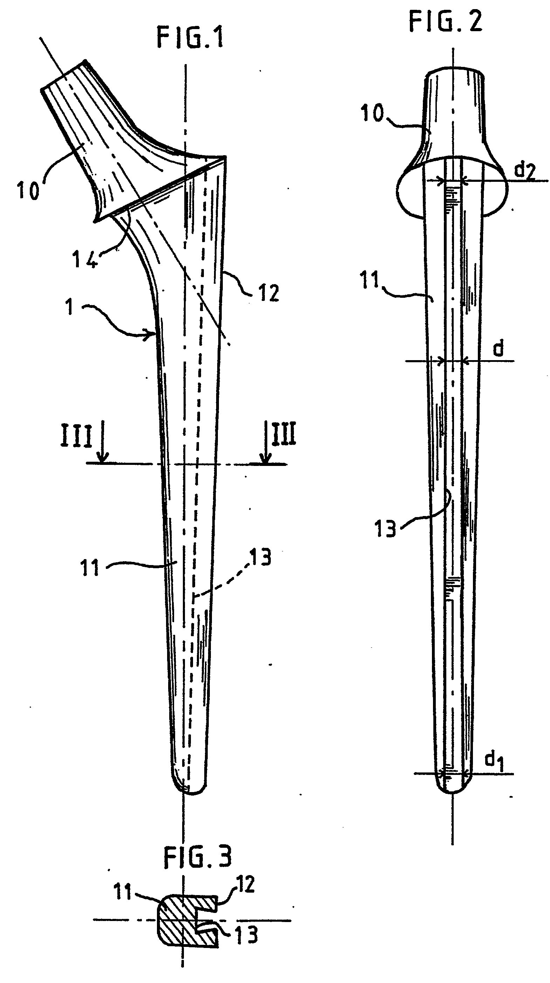

Le corps de prothèse désigné par la référence 1 sur les figures 1 à 3, est une prothèse fémorale, de type général connu, qui présente une tige plate allongée 11, une collerette 14, et un cône morse 10 ; ce dernier est destiné à recevoir, de manière usuelle, une tête fémorale.

Conformément à l'invention, le bord externe 12 du corps 1 a une forme rectiligne ; ce bord est creusé d'une rainure longitudinale 13 de section en queue-d'aronde. La largeur d de cette rainure augmente très légèrement lorsqu'on se déplace de l'extrémité proximale (en haut des figures 1 et 2) vers son extrémité distale (en bas de ces figures) ; la largeur $d_2$ de la rainure à l'éxtrémité proximale est par exemple de l'ordre de 4 mm tandis que la largeur $d_1$ de la rainure à son extrémité distale est de 4,5 mm. La rainure 13 est débouchante aux deux extrémités du corps 1.

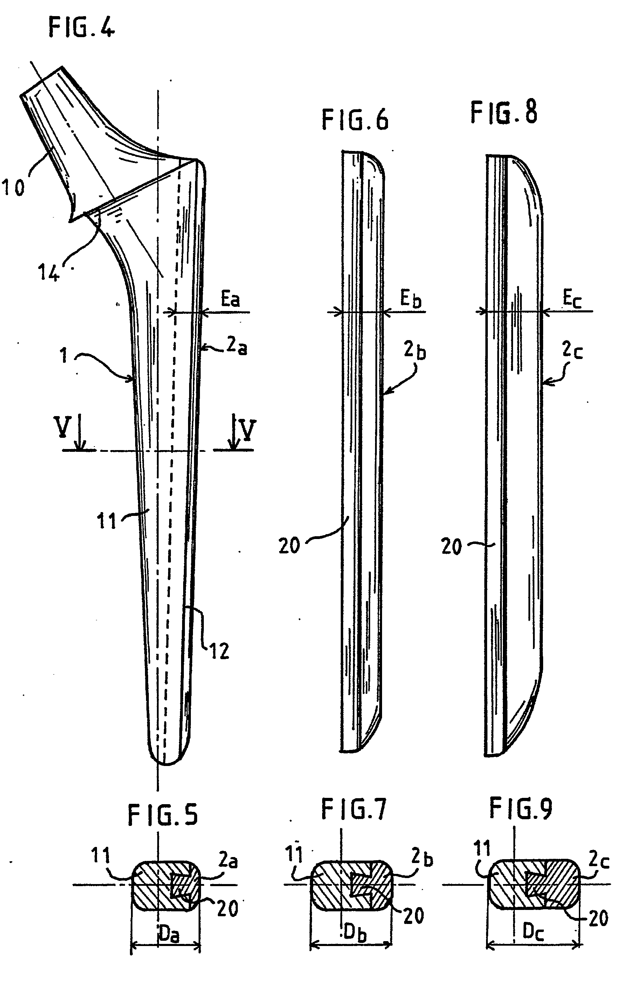

Les figures 4, 6 et 8 représentent trois barrettes modulaires, qui sont destinées à être fixées sélectivement dans le corps de prothèse 1 qui vient d'être décrit.

On a représenté une barrette 2a de faible épaisseur Ea, une barrette 2b d'épaisseur moyenne Eb et une barrette 2c de grande épaisseur Ec. Chacune de ces épaisseurs est sensiblement constante d'une extrémité à l'autre de la barrette considérée. A noter que ces extrémités présentent chacune un bord extérieur courbe, conformé pour se raccorder de manière douce avec les extrémités correspondantes du corps de prothèse.

Chaque barrette 2a, 2b, 2c... , conformément à l'invention, présente un bord intérieur ou une languette 20 identique, en forme de nervure, dont la section en queue-d'aronde est complémentaire de celle de la rainure 13 du corps 1.

A la figure 4, on a représenté la barrette 2a après mise en place dans le corps 1. Cette mise en place de la barrette se fait de bas en haut en raison de la décroissance suivant cette direction de la largeur d de la rainure et de la queue-d'aronde.

La largeur totale du dispositif de prothèse constitué par le corps de prothèse 1 et la barrette 2a, qui a été désignée $D_a$, est par exemple égale à 15 mm au niveau du plan V-V.

Si on utilise au lieu de la barrette 2a la barrette d'épaisseur moyenne 2b, on obtient une épaisseur $D_b$ égale à 20 mm ; de même, si on utilise la barrette 2c, on obtient une largeur totale $D_c$ égale à 25 mm.

Dans un but de simplification, on a représenté simplement trois barrettes modulaires différentes ; il va de soi que dans la pratique, il est souhaitable de prévoir un nombre de barrettes modulaires plus élevé, par exemple neuf barrettes dont les épaisseurs sont échelonnées de millimètre en millimètre.

L'intérêt de l'invention se comprend directement de l'explication qui précède. En effet, en fonction des diamètres du fémur et du canal médullaire dans lequel il doit poser la prothèse (dimensions qu'il peut déduire des radiographies), le chirurgien sélectionne l'une des barrettes de la série qu'il a à sa disposition, met en place cette dernière dans le corps de prothèse, et pose l'ensemble du dispositif dans le canal médullaire à la manière d'une prothèse fémorale traditionnelle.

Il va de soi que, selon les circonstances, le dispositif peut être posé avec ou sans ciment ; dans ce dernier cas, il peut être souhaitable de prévoir à la fabrication du corps de prothèse, dans la partie supérieure de celui-ci, des aspérités telles que des rainures, des nervures, ou un quadrillage, destinés à améliorer la retenue de la prothèse dans le tissu osseux.

Il va de soi que l'invention n'est pas limitée au mode de réalisation préférentiel qui vient d'être décrit à simple titre d'exemple ; elle en embrasse au contraire toutes les variantes.

C'est ainsi qu'il serait possible, bien entendu, de compléter la fixation de la barrette modulaire au corps de prothèse par des moyens additionnels, par exemple à l'aide d'une vis de blocage à tête noyée apte à immobiliser la barrette dans la rainure après son emboîtement de manière à empêcher son coulissement longitudinal.

Par ailleurs, on ne sortirait naturellement pas du cadre de l'invention en inversant les éléments mâle et femelle du système d'assemblage à queue-d'aronde, c'est-à-dire en prévoyant la nervure sur le corps de prothèse et la rainure dans la barrette modulaire.

Le principe de l'invention pourrait être naturellement appliqué à des prothèses autres que des prothèses de la hanche, notamment à des prothèses de l'épaule ou de genou.

**Revendications**

1. Dispositif de prothèse comprenant un corps de prothèse (1) de forme allongée et une série de barrettes modulaires (2a, 2b, 2c...) d'épaisseurs différentes (Ea, Eb, Ec...) adaptées pour constituer avec le corps de prothèse (1) un ensemble de largeur (Da, Db, Dc...) variable, caractérisé par le fait que chacune desdites barrettes (2a, 2b, 2c...) présente une languette (20) apte à être emboîtée dans une rainure (13) de section complémentaire ménagée latéralement dans ledit corps (1), cet emboîtement assurant la solidarisation mutuelle, en direction latérale, du corps (1) avec la barrette (2a, 2b, 2c...).

2. Dispositif de prothèse selon la revendication 1, caractérisé par le fait que chacune desdites barrettes (2a, 2b, 2c...) a une épaisseur (Ea, Eb, Ec...) sensiblement constante de l'une à l'autre de ses extrémités.

3. Dispositif de prothèse selon l'une des revendications 1 ou 2, caractérisé par le fait que la languette est de section en queue-d'aronde (20) et que la rainure est de section complémentaire (13) et que la languette et la rainure ont une largeur (d) qui varie légèrement de l'une à l'autre de leurs extrémités.

4. Dispositif de prothèse selon la revendication 3, caractérisé par le fait que la languette en queue- d'aronde (20) et la rainure (13) ont une largeur (d) qui augmente de l'extrémité proximale en direction de l'extrémité distale.

5. Dispositif de prothèse selon l'une des revendications 1 à 4, destiné à être implanté dans un fémur, caractérisé par le fait que le corps (1) est adapté pour recevoir une tête d'articulation de la hanche.

## Claims

1. Prosthetic device comprising a prosthesis body (1) of elongate shape and a series of modular bars (2a, 2b, 2c ...) of differing thicknesses (Ea, Eb, Ec ...) adapted to make up, with the prosthesis body (1), a unit of variable width (Da, Db, Dc ...), characterized in that each of the said bars (2a, 2b, 2c ...) has a tongue (20) suitable for insertion into a groove (13) of complementary section made in the side of the said body (1), this insertion bringing about the mutual connection in the lateral direction of the body (1) with the bar (2a, 2b, 2c ...).

2. Prosthetic device according to Claim 1, characterized in that each of the said bars (2a, 2b, 2c ...) has a thickness (Ea, Eb, Ec ...) which is substantially constant from one of its ends to the other.

3. Prosthetic device according to either of Claims 1 or 2, charaterized in that the tongue is dovetail-shaped in section (20) and in that the groove is of complementary section (13), and in that the tongue and the groove have a width (d) which varies slightly from one of their ends to the other.

4. Prosthetic device according to Claim 3, characterized in that the dovetail-shaped tongue (20) and the groove (13) have a width (d) which increases from the proximal end towards the distal end.

5. Prosthetic device according to Claim 1 to 4, intended for implantation in a femur, characterized in that the body (1) is adapted to receive a hip joint head.

## Patentansprüche

1. Prothesenvorrichtung, enthaltend einen Prothesenkörper (1) von länglicher Form und eine Reihe von modularen Stäben (2a, 2b, 2c ...) unterschiedlicher Dicken (Ea, Eb, Ec ...), die dazu bestimmt sind, zusammen mit dem Prothesenkörper (1) eine Anordnung variabler Größe (Da, Db, Dc ...) zu bilden, dadurch gekennzeichnet, daß jeder der genannten Stäbe (2a, 2b, 2c ...) eine Feder (20) aufweist, die dazu eingerichtet ist, in eine Nut (13) komplementären Querschnitts eingeführt zu werden, die seitlich in dem genannten Körper (1) ausgebildet ist, wobei diese Einführung die gegenseitige feste Verbindung in Querrichtung des Körpers (1) mit dem Stab (2a, 2b, 2c ...) sicherstellt.

2. Prothesenvorrichtung nach Anspruch 1, dadurch gekennzeichnet, daß jeder der genannten Stäbe (2a, 2b, 2c ...) eine Dicke (Ea, Eb, Ec ...) hat, die vom einen zum anderen Ende des Stabes im wesentlichen konstant ist.

3. Prothesenvorrichtung nach einem der Ansprüche 1 oder 2, dadurch gekennzeichnet, daß die Feder einen schwalbenschwanzförmigen Querschnitt (20) aufweist und daß die Nut einen komplementären Querschnitt (13) hat und daß die Feder und die Nut eine Breite (d) haben, die vom einen zum anderen Ende leicht variiert.

4. Prothesenvorrichtung nach Anspruch 3, dadurch gekennzeichnet, daß die Schwalbenschwanzfeder (20) und die Nut (13) eine Breite (d) haben, die vom naheliegenden Ende in Richtung zum fernliegenden Ende zunimmt.

5. Prothesenvorrichtung nach einem der Ansprüche 1 bis 4, die dazu bestimmt ist, in einen Oberschenkelknochen implantiert zu werden, dadurch gekennzeichnet, daß der Körper (1) dazu eingerichtet ist, einen Hüftgelenkskopf aufzunehmen.

FIG.1

FIG.2

FIG.3

FIG.4

FIG.6

FIG.8

FIG.5

FIG.7

FIG.9